# EUROPEAN PATENT APPLICATION

(11) **EP 4 450 068 A1**
(43) Date of publication of application: **23.10.2024**
(21) Application number: 22908011.4
(22) Date of filing: 16.12.2022
(51) Int. Cl.: A61K 9/51, A61K 47/54, A61K 39/00, A61P 35/00, C07K 16/28

(54) **DENDRITIC CELL-MIMICKING FUNCTIONAL NANOSTRUCTURE COMPRISING alphaCTLA-4 AND METHOD FOR PREPARING SAME**

(30) Priority: 16.12.2021 KR 20210180958; 15.12.2022 KR 20220176122
(71) Applicant: Fortuga Bio Inc., Seoul 04790 (KR)
(72) Inventor: HA, Sang-Jun, Seoul 07986 (KR); MOON, Chae-Won, Gwangmyeong-si Gyeonggi-do 14241 (KR); KIM, Da-Hae, Gimpo-si Gyeonggi-do 10111 (KR); HONG, Jinkee, Seoul 03722 (KR); KIM, Taihyun, Seoul 03722 (KR); LEE, Yoojin, Seoul 03722 (KR)
(74) Representative: Abel & Imray LLP
(86) International application number: PCT/KR2022/020604
(87) International publication number: WO 2023/113546

(57) **Abstract**

The present invention relates to a dendritic cell-mimicking nanostructure for inhibiting immune checkpoints and a method for preparing same, the nanostructure comprising: a nanoparticle core; and a shell including a cell membrane of a lipid molecule derived from a dendritic cell, wherein the shell contains an αCTLA-4 monoclonal antibody. The present invention can provide an excellent effect of immune-anticancer treatment by increasing the immune response by means of strong activation of cytotoxic T cells through the inhibitory activity of the immune checkpoints.

## Description

### [Technical Field]

The present invention relates to a dendritic cell-mimicking functional nanostructure containing αCTLA-4 and a method for producing the same.

### [Background Art]

Cancer ranks first among the causes of death in Korea. A representative cancer treatment method includes surgery to remove cancer, a radiation treatment, and a chemical treatment, but these treatment methods have the problems of poor prognosis and serious side effects.

The main cause of cancer development is tumor formation by cancer cells that cannot be eliminated by the immune response due to decreased immunity or immune evasion of cancer cells. Therefore, an anti-cancer immunotherapeutic agent that can help treat cancer by increasing the patient's own immune response can be a fundamental cancer treatment method. Currently developed anti-cancer immunotherapeutic agents mostly involve direct injection of drugs that enhance immune function, but drug injection still has limitations in that the delivery efficiency is significantly low and additional side effects may exist. Accordingly, studies are focusing on the development of therapeutic agents that can dramatically increase cancer treatment efficiency, reduce recurrence rates and side effects, and increase immune function.

Meanwhile, studies on nano-bio convergence technology that combines nanotechnology with the biomedical field have continued. However, most nanomaterials are obtained through synthesis, and surface modification using nanomaterials involves a process of adding a synthetic substance, which may lead to side effects such as toxicity in the body, induction of unwanted immune responses, and induction of cancer. Therefore, a surface modification method that directly mimics a living body is used, such that side effects may be minimized and various functions may be implemented according to biological characteristics, thereby overcoming the limitations of the existing nanotechnology.

In particular, in a case of a nanoparticle-cellularization technology, which is a technology that physically cloaks a surface of a nanoparticle by using the entire cell membrane of a specific cell as a coating material, complex properties of the cell membrane may be maintained along with proteins, lipids, and carbohydrates, such that the properties of the specific cell may be implemented on the surface of the nanoparticle as they are.

Based on the technology that introduces platelets into a surface of a poly(lactic-co-glycolic) acid (PLGA) particle for the first time in 2015 by Liangfang Zhang's research team at the University of California, the nanoparticle-cellularization technology has been applied to various cells to this day.

Studies have been reported on the removal of solid tumors by producing an immunocompatible nanocarrier by loading doxorubicin onto a PLGA core and then coating the PLGA core with a red blood cell membrane, or the increases in circulation time in the body and tumor targeting ability by producing nanoparticles with hybrid cell membranes by coating melanin nanoparticles with red blood cell membranes and cancer cell membranes at the same time.

However, the conventional cell membrane coating technology was mostly focused on cancer cells and blood cells, and applications thereof were also mainly focused on studies aimed at stable delivery in the blood. In addition, in a case where a cancer cell membrane is directly introduced as an antigen and delivered into a body, there are disadvantages such as decreased immune response due to tolerance to the antigen and resistance to substances derived from cancer cells. Therefore, it is significantly required to develop an immunotherapeutic agent that may have a function of a direct antigen-presenting immune cell and induce differentiation and proliferation of T cells without an intermediate process.

### [Related Art Document]

### [Non-Patent Documents]

(Non-Patent Document 0001) Hu, Che-Ming J., et al. Nature, 2015, 526(7571) 118-12
(Non-Patent Document 0002) Brian T. Luk et al., Theranostics. 2016, 6(7), 1004-1101
(Non-Patent Document 0003) Q. Jiang et al., Biomaterials 2019, 192, 292-308

### [Disclosure]

### [Technical Problem]

An object of the present disclosure is to break away from the material limitations of the related art and to more effectively implement a cancer treatment effect by increasing an immune response by using cells having an antigen-presenting function. Specifically, an object of the present disclosure is to directly activate cytotoxic T cells through inhibition of an immune checkpoint by modifying a surface of a nanostructure that mimics a dendritic cell with a monoclonal antibody αCTLA-4 that may bind to CTLA-4 so as to induce selective killing of cancer cells and increase an immune response, thereby providing an excellent anti-cancer immunotherapy effect.

### [Technical Solution]

In one general aspect, a dendritic cell-mimicking nanostructure for inhibiting an immune checkpoint includes: a nanoparticle core; and a shell including a cell membrane of a lipid molecule derived from a dendritic cell, wherein the shell contains an αCTLA-4 monoclonal antibody.

In an embodiment of the present disclosure, the αCTLA-4 monoclonal antibody may be conjugated to a lipid molecule.

In an embodiment of the present disclosure, the nanostructure may be pulsed with an antigen.

In an embodiment of the present disclosure, the antigen may be a peptide or protein derived from a tumor antigen.

In an embodiment of the present disclosure, the nanostructures may have an average particle size of 20 nm to 50 µm, and specifically, may have an average particle size of 20 nm to 1,000 nm.

In another general aspect, a pharmaceutical composition for treating cancer contains the dendritic cell-mimicking nanostructure for inhibiting an immune checkpoint.

In still another general aspect, a method for producing dendritic cell-mimicking nanostructures for inhibiting an immune checkpoint includes: (S10) purifying cell membranes from dendritic cells; (S20) forming a cell membrane suspension by applying energy to the cell membranes; (S30) obtaining liposomes from the cell membrane suspension; (S40) mixing nanoparticles and the liposomes and then performing filter extrusion to obtain nanoparticles into which the cell membranes are introduced; and (S50) introducing αCTLA-4 monoclonal antibodies conjugated to lipid molecules into the nanoparticles into which the cell membranes are introduced.

In an embodiment of the present disclosure, the method for producing dendritic cell-mimicking nanostructures for inhibiting an immune checkpoint may further include, after step (S40), pulsing an antigen to the nanoparticles into which the cell membranes are introduced.

### [Advantageous Effects]

The dendritic cell-mimicking nanostructure according to the present disclosure introduces an antigen-presenting function to the surface of the nanoparticle, such that the antigen-presenting function of the dendritic cells is preserved, and at the same time, a targeting function and a photothermal function of the nanoparticles are additionally provided, thereby providing an enhanced anti-cancer immunotherapy effect.

The dendritic cell-mimicking nanostructure according to the present disclosure mimics dendritic cells, such that the nanostructure stays in the body for a long period of time and continuously induces proliferation and differentiation of antigen-specific T cells, thereby providing the effect of increasing the immune response.

The dendritic cell-mimicking nanostructure according to the present disclosure has a surface containing a monoclonal antibody αCTLA-4 that may bind to CTLA-4, such that binding to CTLA-4 present on a surface of a cytotoxic T cell may be induced. Through such binding, inhibition of an immune checkpoint is induced by inhibiting the binding of B7-1/2 present in antigen-presenting cells to CTLA-4, and selective killing of cancer cells is induced by significantly activating cytotoxic T cells, such that the immune response is increased, thereby providing an excellent anti-cancer immunotherapy effect.

### [Description of Drawings]

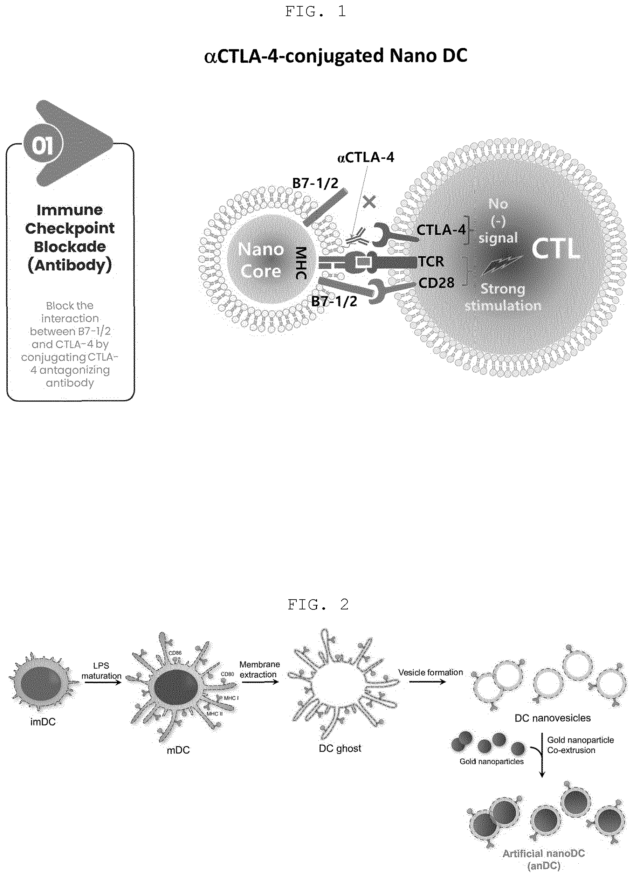

FIG. 1 is a schematic diagram showing a dendritic cell-mimicking nanostructure for inhibiting an immune checkpoint into which an αCTLA-4 monoclonal antibody is introduced (αCTLA-4-conjugated Nano DC), which shows that a cytotoxic T cell may be activated by inhibiting the binding of B7-1/2 to CTLA-4 according to the binding of CTLA-4 of the cytotoxic T cell to αCTLA-4 of the nanostructure.
FIG. 2 is a schematic diagram showing a method for producing dendritic cell-mimicking nanostructures for inhibiting an immune checkpoint according to the present disclosure.
FIG. 3 is a schematic diagram showing a process of pulsing an antigen to the dendritic cell-mimicking nanostructure for inhibiting an immune checkpoint according to the present disclosure to present the antigen on a surface of the nanostructure, inserting an αCTLA-4 conjugated phospholipid into a cell membrane, and finally producing a dendritic cell-mimicking nanostructure for inhibiting an immune checkpoint into which an αCTLA-4 monoclonal antibody is introduced.
FIG. 4 illustrates a method for producing an αCTLA-4 conjugated phospholipid according to the present disclosure.

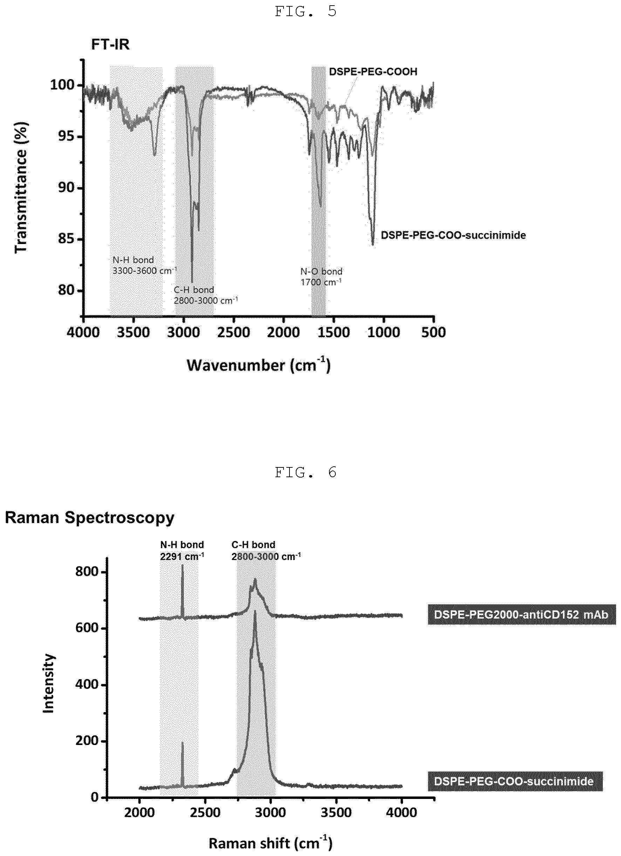

FIG. 5 illustrates the results of FT-IR analysis demonstrating that the αCTLA-4 conjugated phospholipid according to the present disclosure is successfully synthesized.
FIG. 6 illustrates the results of Raman spectroscopy analysis demonstrating that the αCTLA-4 conjugated phospholipid according to the present disclosure is successfully synthesized.

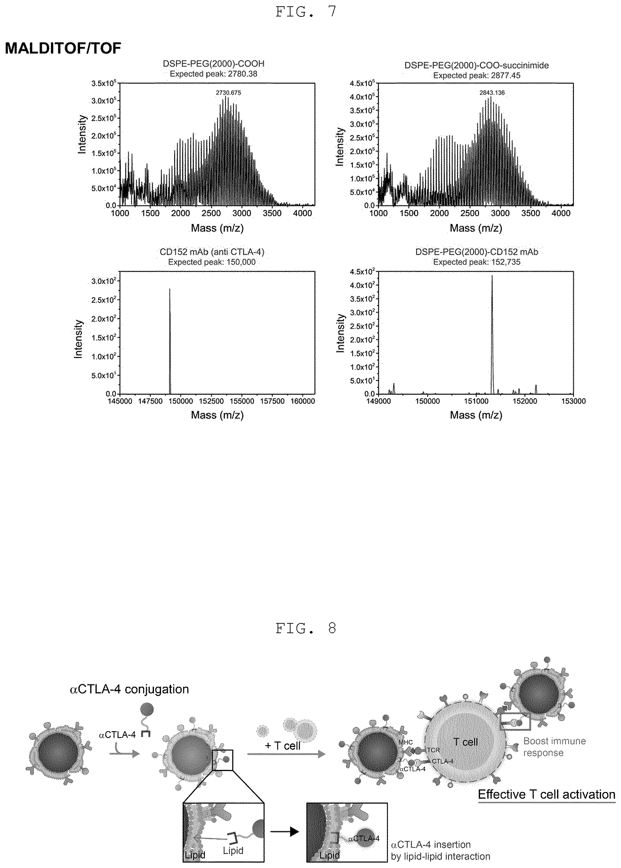

FIG. 7 illustrates the results of MALDI-TOF mass analysis demonstrating that the αCTLA-4 conjugated phospholipid according to the present disclosure is successfully synthesized.
FIG. 8 is a schematic diagram showing the principle of how the dendritic cell-mimicking nanostructure for inhibiting an immune checkpoint into which an αCTLA-4 monoclonal antibody is introduced according to the present disclosure binds to a T cell and activates the immune response.

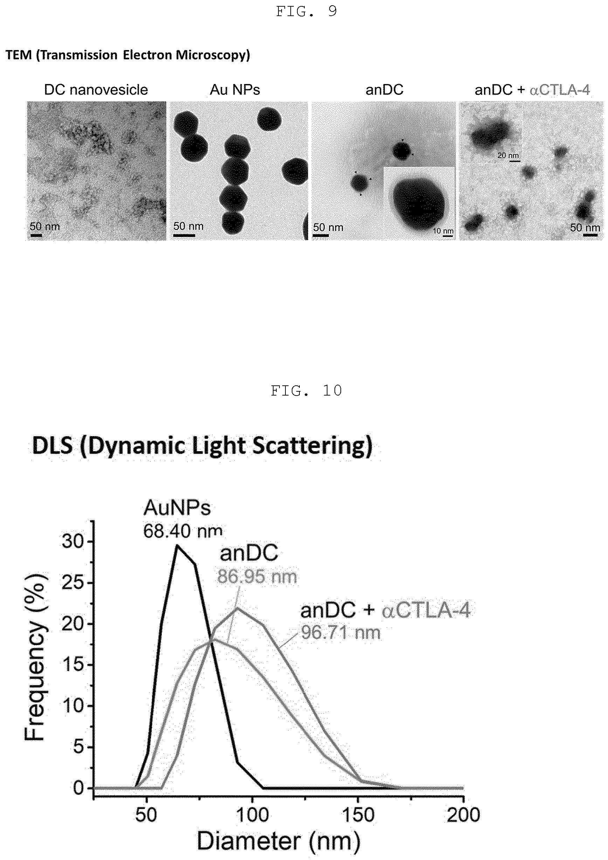

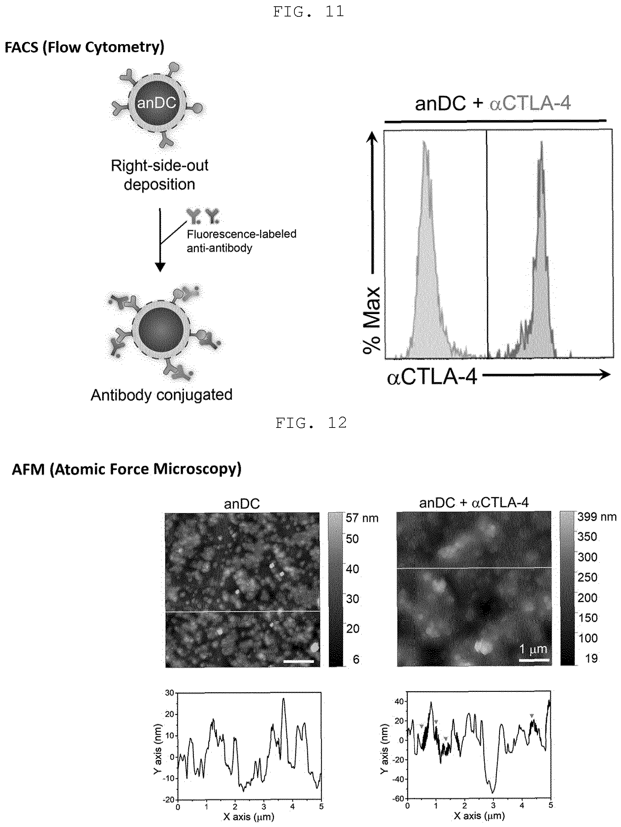

FIGS. 9 to 12 illustrate the results of analyzing a particle size and the like of the dendritic cell-mimicking nanostructure for inhibiting an immune checkpoint into which an αCTLA-4 monoclonal antibody is introduced.
FIGS. 13 to 16 illustrate the results of in vitro and in vivo experiments on T cell proliferation and differentiation inducing effects of the dendritic cell-mimicking nanostructure for inhibiting an immune checkpoint into which an αCTLA-4 monoclonal antibody is introduced according to Experimental Example 1 and Experimental Example 2 of the present disclosure.
FIGS. 17 to 20 illustrate the in vivo experimental process and results on the effect of colon cancer growth inhibition and mouse survival rate of the dendritic cell-mimicking nanostructure for inhibiting an immune checkpoint into which an αCTLA-4 monoclonal antibody is introduced according to Experimental Example 3 of the present disclosure.
FIGS. 21 to 24 illustrate the in vivo experimental process and results on the effect of lung cancer growth inhibition and mouse survival rate of the dendritic cell-mimicking nanostructure for inhibiting an immune checkpoint into which an αCTLA-4 monoclonal antibody is introduced according to Experimental Example 4 of the present disclosure.

### [Best Mode]

Hereinafter, a dendritic cell-mimicking functional nanostructure containing αCTLA-4 and a method for producing the same according to the present invention will be described in detail so that the present invention may easily be implemented by those skilled in the art to which the present invention pertains, with reference to the accompanying drawings and embodiments. However, the present invention may be implemented in various different forms, and the present invention is not limited to embodiments described herein. In addition, the following description is not intended to limit the scope of protection defined by the claims.

Unless otherwise defined, all the technical terms and scientific terms used in the description of the present invention have the general meanings as commonly understood by those skilled in the art to which the present invention pertains, and the description for the known function and configuration unnecessarily obscuring the gist of the present invention will be omitted in the following description.

A numerical range used in the present specification includes upper and lower limits and all values within these limits, increments logically derived from a form and span of a defined range, all double limited values, and all possible combinations of the upper and lower limits in the numerical range defined in different forms. Unless otherwise specifically defined in the specification of the present invention, values out of the numerical range that may occur due to experimental errors or rounded values also fall within the defined numerical range.

Unless the context clearly indicates otherwise, singular forms used in the present specification may be intended to include plural forms.

In the present specification, the expression "comprise(s)" is intended to be an open-ended transitional phrase having an equivalent meaning to "include(s)", "contain(s)", "have (has)", and "are (is) characterized by" and does not exclude elements, materials, or processes, all of which are not further recited herein.

Hereinafter, the present invention will be described in detail.

A dendritic cell-mimicking nanostructure for inhibiting an immune checkpoint according to the present disclosure has a surface containing a monoclonal antibody αCTLA-4 that may bind to CTLA-4, such that binding to CTLA-4 present on a surface of a cytotoxic T cell is induced, and binding of B7-1/2 present in an antigen-presenting cell to CTLA-4 is inhibited, resulting in induction of inhibition of immune checkpoints, thereby providing an effect of significantly activating cytotoxic T cells. The dendritic cell-mimicking nanostructure further induces selective killing of cancer cells and increases an immune response, which exhibits an excellent anti-cancer immunotherapy effect.

To this end, a dendritic cell-mimicking nanostructure for inhibiting an immune checkpoint according to the present disclosure includes a nanoparticle core; and a shell including a cell membrane of a lipid molecule derived from a dendritic cell, wherein the shell contains an αCTLA-4 monoclonal antibody.

CTLA-4 is expressed on cell surfaces of activated CD4+ and CD8+ T cells and is known to be an important negative regulator of T cell function. CTLA-4 has a similar structure to CD28, and binds to ligands CD80/CD86 on dendritic cells with higher intensity and higher affinity than CD28. Therefore, CTLA-4 reduces T cell activation by dendritic cells by reducing the binding potential between CD28 and the ligands.

The dendritic cell-mimicking nanostructure for inhibiting an immune checkpoint according to the present disclosure may further enhance T cell activation through the inhibition of negative regulation by CTLA-4. As an αCTLA-4 antibody, substances known in the art may be used without limitation.

The nanoparticle core may be a one-dimensional or two-dimensional nanostructure. Specific examples of the one-dimensional or two-dimensional nanostructure include, but are not limited to, a carbon nanoribbon, a carbon nanotube, graphene, graphene oxide, reduced graphene oxide, MXene, a metal nanorod, a metal nanowire, and a metal nanoplatelet.

In addition, the nanoparticle core may be one or two or more selected from the group consisting of an organic polymer nanoparticle with biocompatibility, a metal-organic framework nanoparticle, a metal nanoparticle, a metal oxide nanoparticle, a solid lipid nanoparticle, a magnetic nanoparticle, a photothermal conversion nanoparticle, a nucleic acid-containing nanoparticle, and a protein-containing nanoparticle. Specifically, a nanoparticle core that is light-sensitive without toxicity of the nanomaterial itself and may thus be applied to photothermal therapy is more preferable.

In a non-limiting example, the nanoparticle core may be a gold nanoparticle.

In this case, the nanoparticle cores may have an average particle size of 5 to 1,000 nm or 10 to 500 nm. The nanostructures including the nanoparticle core and the shell may have an average particle size of 20 nm to 50 pm, specifically, 20 nm to 10 pm, more specifically, 20 nm to 1,000 nm, and still more preferably 50 nm to 500 nm.

In an example of the present disclosure, the αCTLA-4 monoclonal antibody may be conjugated to a lipid molecule. The lipid molecule may be used without limitation as long as it is a lipid molecule with biocompatibility, specifically, may be a phospholipid, and more specifically, may be a phosphatidylethanolamine-based phospholipid.

According to an example, the phosphatidylethanolamine-based phospholipid and the αCTLA-4 monoclonal antibody may be covalently bonded by a coupling agent. The αCTLA-4 monoclonal antibody covalently bonded to a phospholipid may be easily inserted into the shell including the cell membrane of the lipid molecule by utilizing a spontaneous cell membrane insertion phenomenon based on an interaction with a phospholipid and a lipid bilayer present in the cell membrane, and thus may be stably introduced into the surface of the nanostructure.

Dendritic cells, which are strong antigen-presenting cells, have a cell membrane surface on which a major histocompatibility complex I/II (MHC I/II), co-stimulatory factors (co-stimulatory molecules) such as CD80 and CD86, and cell adhesion molecules such as ICAM-1 are highly expressed, and secrete large amounts of various cytokines, such as interferon, IL-12, and IL-18, which are related to T cell activation, such that the dendritic cells may perform a strong antigen-presenting function. Accordingly, the nanostructure according to the present disclosure may be preferably pulsed with an antigen.

The antigen may be a peptide or protein derived from a tumor antigen for use in a cancer treatment. Specifically, the antigen may be, for example, a protein or peptide derived from ovalbumin (OVA), lymphocytic choriomeningitis mammarenavirus (LCMV) glycoprotein, or retrovirus protein in a mouse cancer model. More specifically, the antigen may be a cancer antigen peptide or protein of OVA257-264, GP33-41, or p15E model.

In addition, in a human cancer antigen, the antigen may be a peptide or protein derived from (1) a tumor-related antigen containing HER2/Neu, tyrosinase, gp100, MART, HPV E6/E7, EBV EBNA-1, carcinoembryonic antigen, a-fetoprotein, GM2, GD2, testis antigen, prostate antigen, and CD20 and (2) a tumor-specific antigen containing a neoantigen that may be produced by various mutations.

As a non-limiting example, the peptide derived from a tumor antigen may be GP₃₃₋₄₁.

The shell of the nanostructure according to the present disclosure may be labeled with a bioactive polymer, a bioactive ingredient, or a protein, in addition to the αCTLA-4 monoclonal antibody.

Specifically, the bioactive ingredient may refer to a cytokine for cell signaling. Non-limiting examples of the bioactive ingredient include chemokines, interferons, interleukins, lymphokines, tumor necrosis factors, monokines, and colony stimulating factors. More specifically, the cytokine may be one selected from the group consisting of a bone morphogenetic protein (BMP) family, a chemokine ligand (CCL) family, a CKLF-like MARVEL transmembrane domain containing member (CMTM) family, a C-X-C motif ligand (CXCL) family, a growth/differentiation factor (GDF) family, a growth hormone, an interferon (IFN) family, an interleukin (IL) family, a tumor necrosis factor (TNF) family, glycophosphatidylinositol (GPI), secreted Ly-6/uPAR-related protein 1 (SLUPR-1), secreted Ly-6/uPAR-related protein 2 (SLUPR-2), and a combination thereof.

The cytokine induces or inhibits transcription factors or growth factors essential for T cell differentiation and mediates the growth and differentiation of other immune cells, such that the anti-cancer immunotherapy effect of the dendritic cell-mimicking structure according to the present disclosure may be further enhanced.

Specifically, the bioactive ingredient is not limited as long as it is an immune checkpoint inhibitor such as αPD-1 or αPD-L1 other than the αCTLA-4 monoclonal antibody, or an anticancer drug such as a chemotherapy drug using the same.

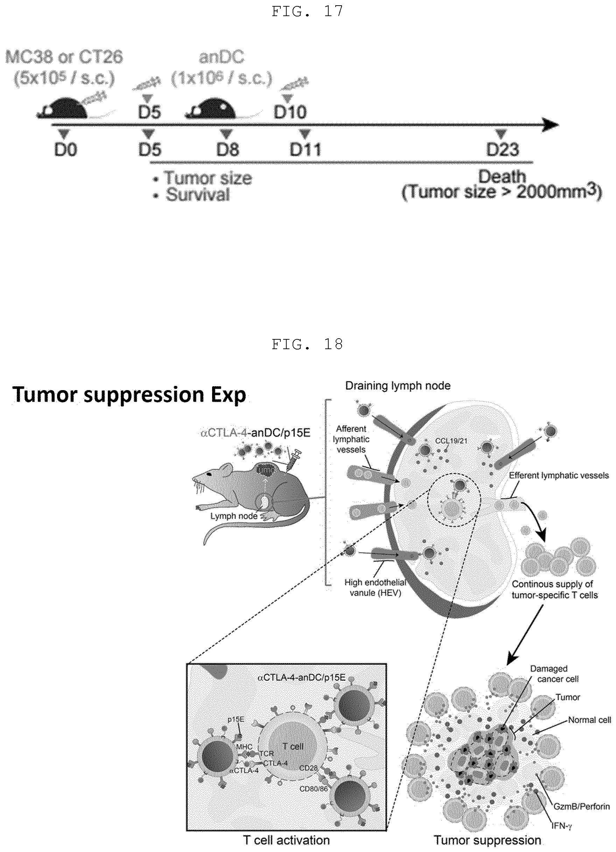

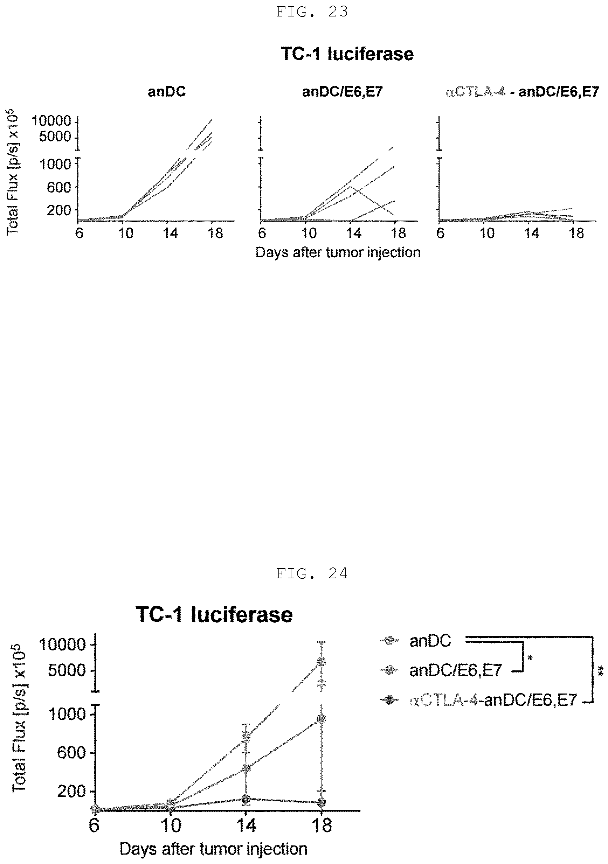

The present disclosure may provide a pharmaceutical composition for treating cancer containing the dendritic cell-mimicking nanostructure for inhibiting an immune checkpoint as described above. Referring to FIGS. 17 to 24, it can be seen that the dendritic cell-mimicking nanostructure for inhibiting an immune checkpoint into which an αCTLA-4 monoclonal antibody is introduced is significantly effective in suppressing tumor growth, and thus exhibits a significantly remarkable effect as a cancer treatment substance. Accordingly, a pharmaceutical composition for treating cancer containing a dendritic cell-mimicking nanostructure for inhibiting an immune checkpoint may provide an excellent anti-cancer immunotherapy effect.

Cancer is a general term for various malignant solid tumors that may expand locally and metastasize by invasion, and specific examples thereof include, but are not limited to, B-cell lymphoma, non-small cell lung cancer, small cell lung cancer, basal cell carcinoma, cutaneous squamous cell carcinoma, colorectal cancer, melanoma, head and neck squamous cancer, hepatocellular carcinoma, stomach cancer, sarcoma, gastroesophageal cancer, renal cell carcinoma, glioblastoma, pancreatic cancer, bladder cancer, prostate cancer, breast cancer, cutaneous T-cell lymphoma, Merkel cell carcinoma, and multiple myeloma.

The pharmaceutical composition for treating cancer may contain formulation substances to modify, maintain, or preserve pH, osmosis, a viscosity, sterility, clarity, color, isotonicity, odor, stability, a dissolution rate or release rate, adsorption, or penetration. The pharmaceutical composition may be administered orally or parenterally. As a parenteral example, the pharmaceutical composition may be administered to a patient by various routes, including intravenous, intramuscular, subcutaneous, intraorbital, intracapsular, intraperitoneal, intrarectal, intracisternal, intravascular, and intradermal routes, and may also be administered through the skin using a skin patch or transdermal iontherapy.

The pharmaceutical composition may be administered as an individual therapeutic agent or in combination with another anticancer agent, and may be administered sequentially or simultaneously with a conventional anticancer agent. In addition, the pharmaceutical composition may be administered singly or multiple times. Considering all the factors, it is important to administer the pharmaceutical composition in an amount capable of obtaining a maximum effect with a minimal amount without side effects, and the amount of the pharmaceutical composition to be administered may be easily determined by those skilled in the art.

In addition, according to the present disclosure, it is possible to provide a method for treating cancer in a subject, the method including administering the dendritic cell-mimicking nanostructure for inhibiting an immune checkpoint or a pharmaceutical composition containing the same to a subject suffering from cancer. The pharmaceutical composition is administered to a subject, such that a tumor-specific immune response may be induced in the subject, or symptoms of cancer may be treated and/or alleviated in the subject. In this case, the subject may be a human or non-human mammal.

In addition, the present disclosure provides a method for producing dendritic cell-mimicking nanostructures for inhibiting an immune checkpoint, the method including: (S10) purifying cell membranes from dendritic cells; (S20) forming a cell membrane suspension by applying energy to the cell membranes; (S30) obtaining liposomes from the cell membrane suspension; (S40) mixing nanoparticles and the liposomes and then performing filter extrusion to obtain nanoparticles into which the cell membranes are introduced; and (S50) introducing αCTLA-4 monoclonal antibodies conjugated to lipid molecules into the nanoparticles into which the cell membranes are introduced.

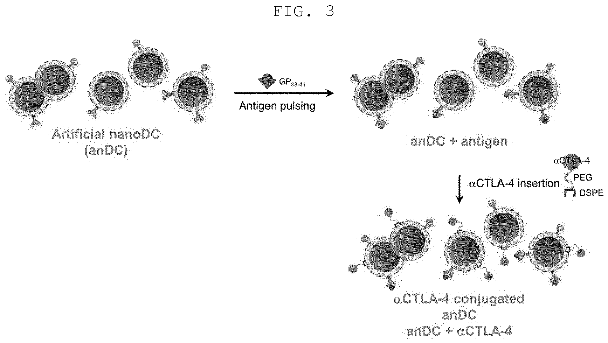

Referring to FIG. 3, a process of pulsing an antigen to a dendritic cell-mimicking nanostructure (anDC) to present the antigen on a surface of the nanostructure, inserting an αCTLA-4 conjugated phospholipid into a cell membrane, and finally producing a dendritic cell-mimicking nanostructure for inhibiting an immune checkpoint into which an αCTLA-4 monoclonal antibody is introduced is illustrated.

Specifically, (S10) the purifying of the cell membranes from the dendritic cells may be performed by rapid freezing-thawing and centrifugation. (S20) A cell membrane suspension may be formed by applying energy to the purified cell membranes. In this case, as an example, when the cell membranes are sonicated, a cell membrane suspension in hundreds of nanoscale or several microscale sizes may be obtained. Thereafter, (S30) when the cell membrane suspension is filtered through a membrane filter, cell membrane liposomes of a desired size may be obtained. (S40) The cell membrane liposomes and nanoparticles may be mixed and subjected to filter extrusion to obtain nanoparticles into which cell membranes are introduced. A form in which the cell membrane of the dendritic cell is coated on the surface of the nanoparticle is obtained.

The method may further include, after step (S40), pulsing an antigen to the nanoparticles into which the cell membranes are introduced. The antigen may be a peptide or protein derived from a tumor antigen for use in a cancer treatment, and various tumor-specific antigens may be used as described above. In the present disclosure, as a non-limiting example, GP₃₃₋₄₁ may be used.

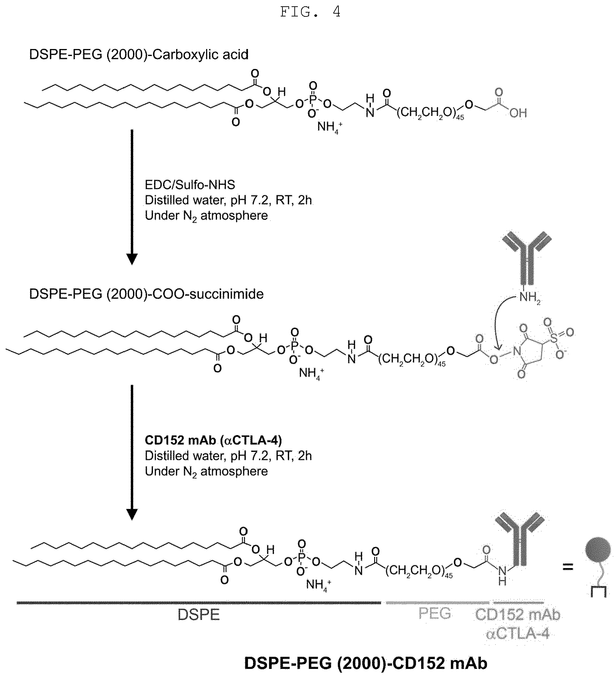

In step (S50), the lipid molecule may be used without limitation as long as it is a lipid molecule with biocompatibility, specifically, may be a phospholipid, and more specifically, may be a phosphatidylethanolamine-based phospholipid. Specific examples of the phosphatidylethanolamine-based phospholipids include 2-distearoyl-sn-glycero-3-phosphoethanolamine (DSPE). FIG. 4 illustrates a process of obtaining αCTLA-4 conjugated to a lipid molecule. One end of PEG may be conjugated to a hydrophilic group of DSPE, and αCTLA-4 may be conjugated to the other end of PEG. A weight average molecular weight of PEG may be 200 to 10,000 g/mol or 500 to 5,000 g/mol.

αCTLA-4 conjugated to a phospholipid may be easily inserted according to lipid-lipid interactions in the cell membrane of the dendritic cell nanostructure, effectively inducing the activation of T cells.

Hereinabove, although the present invention has been described by specific matters and limited embodiments, they have been provided only for assisting in the entire understanding of the present invention. Therefore, the present invention is not limited to the embodiments. Various modifications and changes may be made by those skilled in the art to which the present invention pertains from this description.

Therefore, the spirit of the present invention should not be limited to the described embodiments, but the claims and all modifications equal or equivalent to the claims are intended to fall within the spirit of the present invention.

### Production Example 1. Production of Dendritic Cell-Mimicking Nanostructure

Dendritic cells were differentiated from bone marrow cells isolated from the femurs of a 6- to 8-week-old Naive C57BL/6 mouse (Orient Bio), and centrifuged at 2,000 rpm for 5 minutes, thereby obtaining pure dendritic cells. Thereafter, the pure dendritic cells were treated with Protease Inhibitor Tablet-PBS buffer and dispersed at a concentration of 1 to 2 × 10⁶ cells/ml. Then, only the cell membranes were purified through rapid freezing at -70°C, thawing at room temperature, and centrifugation. Thereafter, the cell membranes were treated with ultrasonic waves (VC505, Sonics & Materials) at an amplitude of 20% by performing a total of 60 cycles of 3 seconds each with 3 seconds on/off and a 2 second cooling period between cycles to obtain a micro-scale cell membrane suspension, and the cell membrane suspension was filtered through a polycarbonate membrane with a nano-sized filter (pore size of 1 pm, 400 nm, or 100 nm) to produce nanoliposomes having a diameter of each pore size. Considering a surface area of the cell membrane coated on the nanoparticle surface, it was assumed that X_{DM} = 1 for the case where nanoparticles and liposomes have an optimized mixing ratio.Mole fraction (XDM) = (Surface area of cell membrane) / (Surface area of nanoparticle)

5 µl of 60 nm gold nanoparticles and 1 ml of liposomes were mixed so that X_{DM} = 1, and then subjected to filter extrusion together to produce a dendritic cell-mimicking nanostructure. The produced dendritic cell-mimicking nanostructure was treated with 0.2 pg/ml of GP-33 antigen at 37 °C for about 30 minutes to induce the antigen to be presented on MHC class I and II surfaces, thereby producing a dendritic cell-mimicking nanostructure pulsed with an antigen.

### Example 1. Production of Dendritic Cell-Mimicking Nanostructure for Inhibiting Immune Checkpoint into Which αCTLA-4 Monoclonal Antibody is Introduced

According to the method illustrated in FIG. 4, a molecule in which a PEG end was conjugated to a hydrophilic group of 2-distearoyl-sn-glycero-3-phosphoethanolamine (DSPE) was prepared by conjugating an αCTLA-4 monoclonal antibody to the other end of PEG, and the αCTLA-4 monoclonal antibody conjugated to a phospholipid was introduced into the cell membrane of the dendritic cell-mimicking nanostructure produced according to Production Example 1 using a spontaneous cell membrane insertion phenomenon, thereby producing a dendritic cell-mimicking nanostructure for inhibiting an immune checkpoint.

### Experimental Example 1. Evaluation of T Cell Proliferation and Differentiation Inducing Effects of Dendritic Cell-Mimicking Nanostructure for Inhibiting Immune Checkpoint (In Vitro)

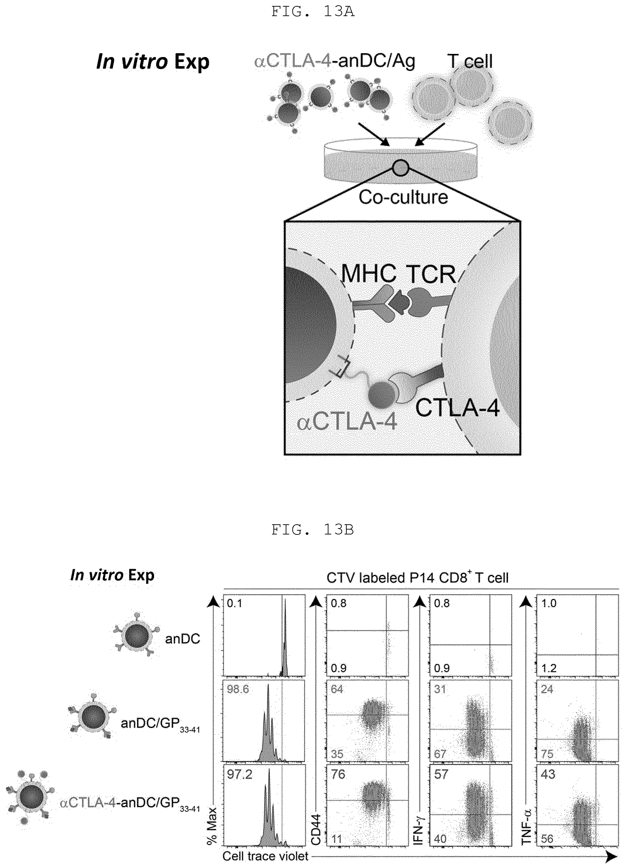

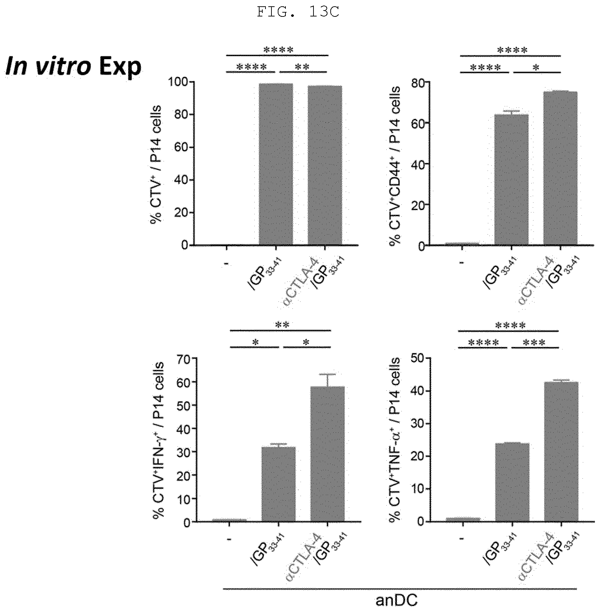

For each of 4 × 10⁴ dendritic cell-mimicking nanostructures for inhibiting an immune checkpoint produced according to Example 1 (αCTLA4-anDC), non-antigen pulsed dendritic cell-mimicking nanostructures (anDC), and antigen-pulsed dendritic cell-mimicking nanostructures (anDC/GP₃₃₋₄₁), in a 96-well U bottom plate, 2 × 10⁵ CD8 T cells isolated from a P14 mouse were added to the same well with 200 µl of RPMI-containing 10% FBS culture medium, and then co-culture was performed at 37 °C for 3 days. 3 days later, the plate was recovered from the incubator, CD8 T cells existing in that plate were stained with a fluorescent antibody , and a degree of activation of CD8 T cells was analyzed using a flow cytometer. The results are illustrated in FIGS. 13B and 13C.

FIG. 13B is a graph of FACS data. In the case of the dendritic cell-mimicking nanostructure for inhibiting an immune checkpoint (αCTLA4-anDC) produced according to Example 1, the highest expression rates were observed for all CD44, IFNγ, and TNFα. Through this, it can be confirmed that the proliferation and activation effects of T cells of the dendritic cell-mimicking nanostructure for inhibiting an immune checkpoint according to the present disclosure are significantly excellent.

### Experimental Example 2. Evaluation of T Cell Proliferation and Differentiation Inducing Effects of Dendritic Cell-Mimicking Nanostructure for Inhibiting Immune Checkpoint (In Vivo)

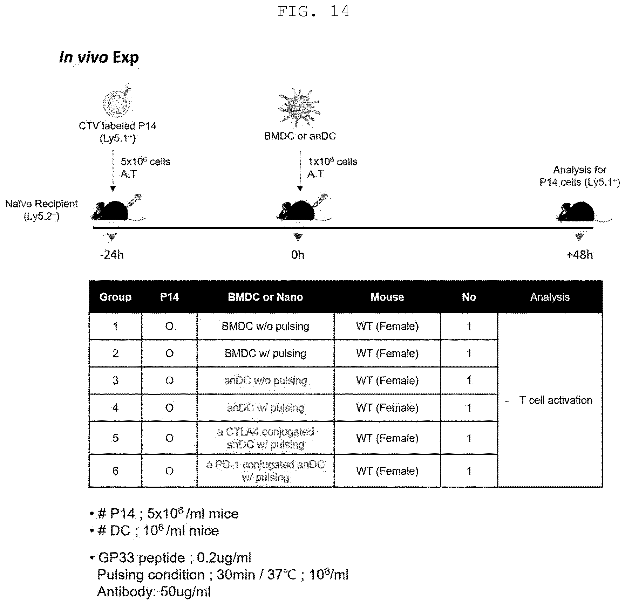

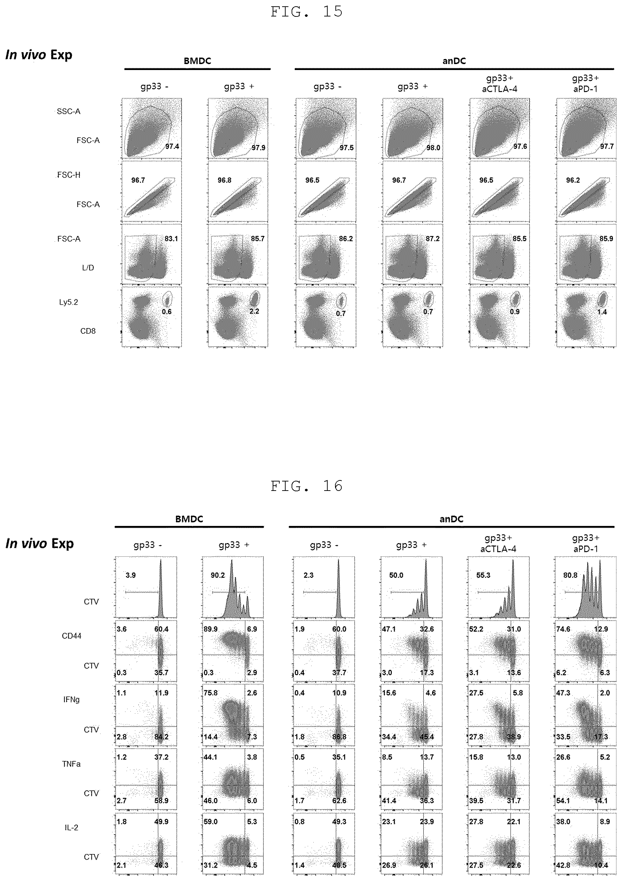

CD8 T cells were isolated from a P14 mouse with a congenic marker (Ly 5.1) and then subjected to adoptive transfer to a naive recipient mouse, and after 24 hours, adoptive transfer was performed on a dendritic cell (BMDC), a dendritic cell-mimicking nanostructures (anDC), and a dendritic cell-mimicking nanostructure for inhibiting an immune checkpoint (αCTLA4 conjugated anDC, αPD-1 conjugated anDC) under different conditions for each group. The specific conditions are illustrated in FIG. 14. After 48 hours, immune cells were isolated from the spleen of the mouse, stained with a fluorescent antibody, and a degree of CD8 T cell activation was analyzed using a flow cytometer. The results are illustrated in FIGS. 15 and 16.

Referring to FIGS. 15 and 16, in the case of Group 3, which was not treated with GP-33, it can be confirmed that, like Group 1, a negative control, all CD8 T cell activation indicators did not appear. In Groups 4 to 6, proliferation of CD8 T cells through CTV, activation through CD44, and secretion of functional cytokines such as IFNγ, TNFα, and IL-2 were confirmed. It can be confirmed in vivo that the proliferation and activation effects of T cells of the dendritic cell-mimicking nanostructure for inhibiting an immune checkpoint according to the present disclosure are excellent.

### Experimental Example 3. Evaluation of Inhibition of Cancer Growth and Mouse Survival Rate to Verify Anti-Cancer Efficacy of Dendritic Cell-Mimicking Nanostructure for Inhibiting Immune Checkpoint into Which αCTLA-4 Monoclonal Antibody is Introduced in Mouse Colon Cancer Model

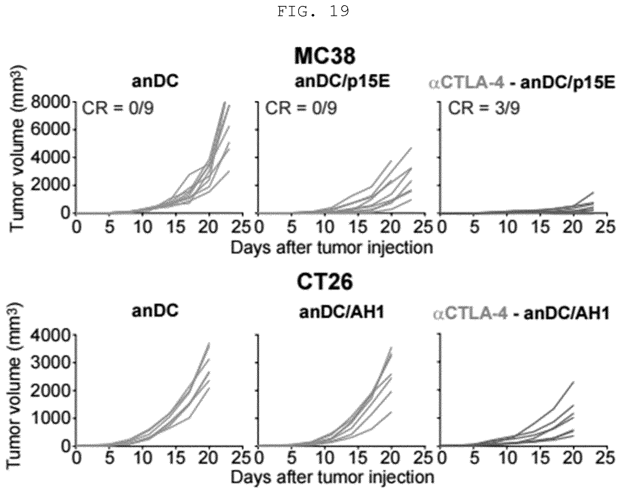

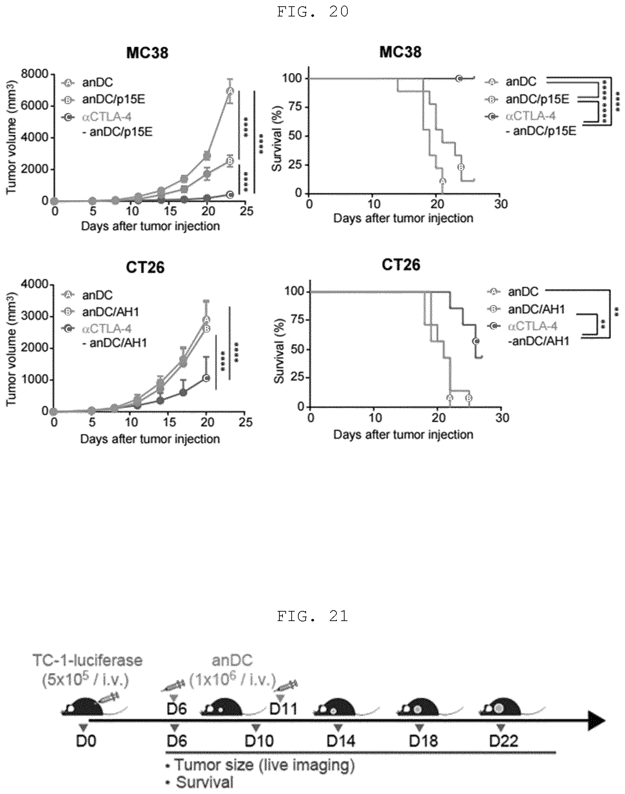

The in vivo test conditions for evaluating the anticancer efficacy of the nanostructure in a mouse colon cancer model are as illustrated in FIG. 17. Using mouse colon cancer cell line MC38 or CT26, tumors were induced by subcutaneously injecting 5 × 10⁵ cells to 6- to 8-week-old C57BL/6 or BALB/c mice respectively. The tumor size was measured using calipers on days 0, 5, 8, 11, 14, 17, 20, and 23, and tumors larger than 2,000 mm³ were considered dead and a survival graph was drawn. 5 and 10 days after tumor cell injection, anDC, anDC/p15E, and αCTLA4-anDC/p15E were injected to each group through subcutaneous injection. The results thereof are illustrated in FIGS. 19 and 20. Referring to FIGS. 19 and 20, in the group injected with 1 × 10⁶ dendritic cell nanostructures into which an αCTLA-4 monoclonal antibody was introduced according to the present disclosure, a significant reduction in tumor growth was confirmed compared to the group injected only with anDC nanostructures, and in addition, it was confirmed that the mouse survival rate was significantly increased.

### Experimental Example 4. Evaluation of Cancer Growth Rate Inhibition to Verify Anti-Cancer Efficacy of Dendritic Cell-Mimicking Nanostructure for Inhibiting Immune Checkpoint into Which αCTLA-4 Monoclonal Antibody is Introduced in Mouse Lung Cancer Model

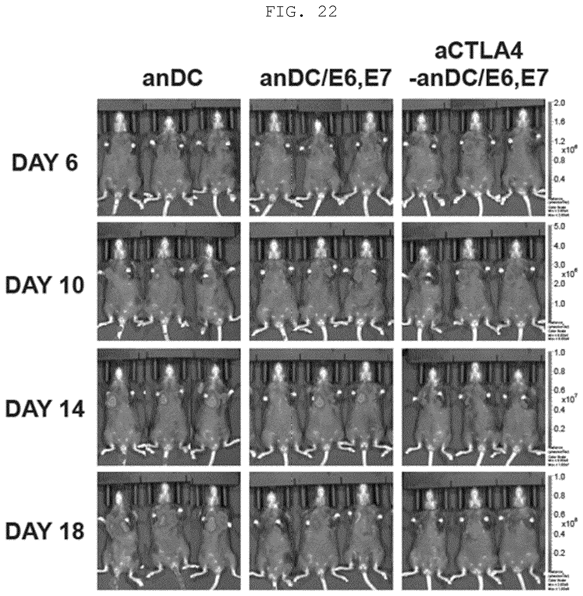

The in vivo test conditions for evaluating the anticancer efficacy of the nanostructure in a mouse lung cancer model are as illustrated in FIG. 21. TC1-luciferase cell line obtained by synthesizing luciferase in the mouse lung cancer cell line TC1, which is an immune checkpoint inhibitor-resistant cell line, was used. Tumors were induced orthotopically by injecting 5 × 10⁵ cells to the tail vein of a 6- to 8-week-old C57BL/6 mouse. After 6 days, tumor formation was confirmed through In Vivo Imaging System (IVIS) spectrum. From day 6, when the tumor was properly formed, anDC, anDC/E6,E7, and αCTLA4-anDC/E6,E7 were injected to three mice per group through tail vein injection at 5-day intervals, and IVIS images were obtained on days 10, 14, and 18. The results thereof are illustrated in FIGS. 22 to 24.

Referring to FIGS. 22 to 24, it can be confirmed that the tumor formation rate was the slowest in the group injected with 1 × 10⁶ dendritic cell-mimicking nanostructures for inhibiting an immune checkpoint into which an αCTLA-4 monoclonal antibody was introduced according to the present disclosure.

## Claims

1. A dendritic cell-mimicking nanostructure for use in inhibiting an immune checkpoint, the dendritic cell-mimicking nanostructure comprising:
a nanoparticle core; and
a shell including a cell membrane of a lipid molecule derived from a dendritic cell,
wherein the shell contains an αCTLA-4 monoclonal antibody.

2. The dendritic cell-mimicking nanostructure of claim 1, wherein the αCTLA-4 monoclonal antibody is conjugated to a lipid molecule.

3. The dendritic cell-mimicking nanostructure of claim 1, wherein the nanostructure is pulsed with an antigen.

4. The dendritic cell-mimicking nanostructure of claim 1, wherein the antigen is a peptide or protein derived from a tumor antigen.

5. The dendritic cell-mimicking nanostructure of claim 1, wherein the nanostructures have an average particle size of 20 nm to 50 µm.

6. The dendritic cell-mimicking nanostructure of claim 1, wherein the nanostructures have an average particle size of 20 nm to 1,000 nm.

7. The dendritic cell-mimicking nanostructure of claim 1, wherein a surface of the shell is labeled with a bioactive polymer, a bioactive ingredient, or a protein.

8. A pharmaceutical composition for use in treating cancer, comprising the dendritic cell-mimicking nanostructure for inhibiting an immune checkpoint of claim 1.

9. A method for producing dendritic cell-mimicking nanostructures for inhibiting an immune checkpoint, the method comprising:
(S10) purifying cell membranes from dendritic cells;
(S20) forming a cell membrane suspension by applying energy to the cell membranes;
(S30) obtaining liposomes from the cell membrane suspension;
(S40) mixing nanoparticles and the liposomes and then performing filter extrusion to obtain nanoparticles into which the cell membranes are introduced; and
(S50) introducing αCTLA-4 monoclonal antibodies conjugated to lipid molecules into the nanoparticles into which the cell membranes are introduced.

10. The method of claim 9, further comprising, after step (S40), pulsing an antigen to the nanoparticles into which the cell membranes are introduced.
